# EUROPÄISCHE PATENTSCHRIFT

(11) **EP 3 189 820 B1**
(45) Veröffentlichungstag und Bekanntmachung des Hinweises auf die Patenterteilung: **14.03.2018**
(21) Anmeldenummer: 16150730.6
(22) Anmeldetag: 11.01.2016
(51) Int. Cl.: A61F 13/00, A61F 13/02

(54) **VERBANDANORDNUNG UND VERFAHREN ZUM BEHANDELN EINER WUNDE**
DRESSING ASSEMBLY AND METHOD FOR TREATING A WOUND
SYSTEME DE PANSEMENT ET PROCEDE DE TRAITEMENT D'UNE PLAIE

(43) Veröffentlichungstag der Anmeldung: 12.07.2017
(73) Patentinhaber: Sefar AG, 9410 Heiden (CH)
(72) Erfinder: GERDES, Gerd, 9030 Abtwil (CH); WEBER, Jérémie, 6833 Klaus (AT)
(74) Vertreter: Wunderlich, Rainer

(56) Entgegenhaltungen:
- EP-A1- 2 959 871
- WO-A1-2006/048246
- WO-A1-2006/056294
- WO-A1-2010/011434
- WO-A1-2010/027544
- WO-A2-2007/030598
- GB-A- 2 468 905

## Beschreibung

Die Erfindung betrifft eine Verbandanordnung zum Behandeln einer Wunde eines menschlichen oder tierischen Körpers mittels Unterdruck, mit einem Absorptionskörper zum Absorbieren von Wundsekret, wobei der Absorptionskörper zum Einsetzen in einen Hohlraum der Wunde ausgebildet ist, einem Trennelement, welches zwischen der Wunde und dem Absorptionskörper angeordnet ist, einem Abdeckelement, welches zum weitgehend luftdichten Abdecken der Wunde mit dem Absorptionskörper ausgebildet ist, und eine Anschlusseinrichtung, welche zum Anschließen einer Unterdruckquelle an dem Abdeckelement angeordnet ist, gemäß dem Oberbegriff des Anspruchs 1.

Es sind aus der WO 2007/030598 A2 eine gattungsgemäße Verbandanordnung und ein Verfahren zum Behandeln einer Wunde eines menschlichen oder tierischen Körpers bekannt, wobei ein Absorptionskörper zum Absorbieren von Wundsekret in einen Hohlraum der Wunde eingesetzt wird, zwischen der Wunde und dem Absorptionskörper ein Trennelement angeordnet ist, die Wunde mit dem eingesetzten Absorptionskörper mittels eines Abdeckelementes weitgehend luftdicht abgedeckt wird und über eine Anschlusseinrichtung an dem Abdeckelement eine Unterdruckquelle angeschlossen wird, mit welcher in der abgedeckten Wunde ein Unterdruck eingestellt wird. Als Trennelement wird eine Gaze verwendet, also ein grobmaschiges Baumwollgewebe.

Eine weitere derartige Verbandanordnung sowie ein derartiges Verfahren gehen beispielsweise aus der WO 2006/048246 A1 hervor. Bei dieser sogenannten Unterdrucktherapie können bei Wunden mit größeren Hohlräumen zuverlässig Wundsekrete abgeführt werden, ohne dass diese hierbei das gesunde Gewebe nachhaltig schädigen können. Der Unterdruck bewirkt, dass Wundsekrete in den Absorptionskörper eindringen. Die Wundsekrete können hierin aufgenommen werden oder mittels einer Abführleitung aus dem Absorptionskörper und der abgedeckten Wunde insgesamt abgeleitet werden.

Es ist bekannt, dass der Absorptionskörper üblicherweise aus einem Schaumstoffmaterial gebildet ist. Dieses Schaumstoffmaterial wird unmittelbar auf die Größe der Wunde zugeschnitten, so dass der Absorptionskörper den Hohlraum der Wunde weitgehend passend ausfüllt, jedoch nicht mit den angrenzenden Hautbereichen in Kontakt gerät. Der aus Schaumstoffmaterial gebildete Absorptionskörper wird üblicherweise mit einem gewissen Abstand zum Wundrand ausgebildet, um so eine Reizung der Haut durch das Schaumstoffmaterial sowie im Absorptionskörper aufgenommenes Wundsekret zu vermeiden.

Des Weiteren ist es bekannt, ein Folienelement als eine Trennschicht zwischen der Wunde und dem Absorptionskörper vorzusehen, um ein Einwachsen des Absorptionskörpers in das Gewebe zu vermeiden. Eine entsprechende Verbandanordnung geht aus der US 2008/0177253 A1 hervor.

In der GB 2468905 A ist ein Verbandmaterial für ein Unterdruckverfahren beschrieben, bei welchem der Absorptionskörper aus einem dreidimensionalen Gewirk hergestellt ist. Zusätzlich kann das dreidimensionale Gewirk von einer perforierten Kunststofffolie oder einem nicht-gewebten Material umhüllt sein.

Aus der EP 2 959 871 A1 geht ein Verbandprodukt hervor, welches in einer versiegelten Kammer gelagert ist.

Der Erfindung liegt die **Aufgabe** zugrunde, eine Anordnung zum Behandeln einer Wunde anzugeben, mit welchem eine besonders schonende Wundbehandlung ermöglicht wird.

Die Aufgabe wird zum einen durch eine Verbandanordnung gemäß dem Anspruch 1 gelöst. Bevorzugte Ausführungsformen der Erfindung sind in den abhängigen Ansprüchen angegeben.

Bei der erfindungsgemäßen Verbandanordnung ist vorgesehen, dass das Trennelement größer als die zu behandelnde Wunde ausgebildet ist, wobei ein überstehender Randabschnitt gebildet ist, und dass der Randabschnitt auf einen Hautbereich am Rand der Wunde angebracht ist.

Eine Grundidee der Erfindung kann darin gesehen werden, dass gegen ein Einwachsen des Absorptionskörpers das vorgesehene Trennelement gezielt größer als der Absorptionskörper und die Wunde insgesamt ausgebildet ist, so dass hierdurch auch der umgebende Hautbereich am Rand der Wunde mit abgedeckt wird. Somit wird ein besonders guter Schutz der Wunde gegen Verschmutzung und des Wundrandes gegen Reizung erzielt.

Gemäß einer Erkenntnis der Erfindung wird der Absorptionskörper vom behandelnden Personal üblicherweise unmittelbar im Nahbereich der Wunde auf die geforderte Größe zugeschnitten. Dabei wird das Schaumstoffmaterial des Absorptionskörpers unmittelbar über die Wunde gehalten und mit einer Schere zurechtgeschnitten. Dabei besteht die Gefahr, dass kleinere Partikel des Absorptionskörpers in die Wunde gelangen und dort zu Schäden, Entzündungen oder Schmerzen für den Patienten führen können.

Des Weiteren besteht die Gefahr, dass der Absorptionskörper in unerwünschter Weise unmittelbar den Wundrand und den angrenzenden Hautbereich kontaktiert. Dies kann zu einer Reizung und einer sogenannten Mazeration des betroffenen Gewebebereiches führen. Dies erschwert und verkompliziert die Wundheilung. Durch das Trennelement, welches auch den Wundrand mit abdeckt, wird dies verhindert, selbst wenn die Absorptionskörper aus dem Wundhohlraum überstehen.

Gemäß der Erfindung wird zu Beginn ein Trennelement großflächig auf die Wunde aufgebracht, wobei durch einen überstehenden Randabschnitt auch ein Hautbereich am Wundrand mit abgedeckt und geschützt wird. Damit kann schon beim Zuschneiden des Absorptionskörpers ein Schutz gegen Verschmutzung oder unerwünschten Wundkontakt erreicht werden. Dabei kann vorzugsweise auch ein Absorptionselement verwendet werden, welches über den Wundrand hinaus ragt. Es können so vorgeformte Standard-Absorptionselemente ohne Zuschneiden eingesetzt werden.

Eine bevorzugte Ausführungsform der Erfindung besteht darin, dass an dem Randabschnitt zumindest bereichsweise eine Haftschicht zum Ankleben des Trennelementes auf den Hautbereich angeordnet ist. Die Haftschicht kann vorzugsweise einen dermatologischen Acryl- oder Silikonkleber aufweisen. Bevorzugt ist es dabei nach der Erfindung, dass das Trennelement eine rechteckige oder längliche Form aufweist, wobei sich insgesamt zwei streifenartige Haftschichten entlang den beiden Längsseiten des Trennelementes erstrecken. Dabei muss das Trennelement nicht allseitig um den Wundrand aufgeklebt werden, sondern kann so nur an zwei gegenüberliegenden Seiten der Wunde mittels der Haftschicht befestigt werden. Die Haftschicht gewährleistet eine einfache und sichere Handhabung.

Besonders vorteilhaft ist es nach der Erfindung, dass das Trennelement ein Gewebe umfasst. Ein Gewebe ist besonders luftdurchlässig und wundschonend.

Ein besonders wirksames Trennelement wird nach der Erfindung dadurch erzielt, dass das Gewebe aus Monofilamentfäden mit einer glatten Oberfläche gebildet ist, welche einen Fadendurchmesser zwischen 10 bis 100 µm, insbesondere zwischen 30 bis 50 µm aufweisen. Die Monofilamentfäden können dabei aus Polyamid, Polyester, Polypropylen oder einem anderen geeigneten Kunststoffmaterial gefertigt sein. Dabei hat sich herausgestellt, dass Polyester (PET) besonders wundschonend ist und einem Verwachsen entgegenwirkt. Eine mögliche Erklärung kann darin gesehen werden, dass PET-Monofilamentfäden eine besonders glatte Oberfläche haben, was einem Anhaften von Proteinen, menschlichen Zellen und Geweben entgegenwirkt.

Nach der Erfindung ist es vorgesehen, dass das Trennelement biokompatibel und antihaftend ausgebildet ist. Dies kann dadurch erzielt werden, dass die Monofilamentfäden mit einer entsprechenden biokompatiblen oder antihaftenden Beschichtung, etwa PTFE, versehen sind oder das Trennelement insgesamt mit einer solchen Schicht versehen ist. Durch eine entsprechende Materialauswahl und/oder Beschichtung kann zudem eine nicht-sensitisierende oder hypoallergene Eigenschaft eingestellt werden.

Eine besonders gute Wirkung des Trennelementes wird nach der Erfindung dadurch erzielt, dass das Gewebe mit einer Maschenweite von 40 bis 500 µm, vorzugsweise 70 bis 300 µm vorgesehen ist. Die Maschenweite bezieht sich dabei auf Gewebe. Bei einem Gewebe ist bevorzugt ein Einfachgewebe, insbesondere mit einer Leinwand-, Köper- oder Atlas-Bindung vorgesehen. Die Gewebedicke beträgt dabei zwischen 20 bis 200 µm, bevorzugt zwischen 50 bis 100 µm.

Gemäß einer anderen Ausführungsform der Erfindung ist es bevorzugt, dass das Abdeckelement größer als das Trennelement ausgebildet ist. Das Abdeckelement ist insbesondere eine luftdichte Folie, welche gegebenenfalls mit einem Gewebematerial verstärkt sein kann. Das Abdeckelement überdeckt so nicht nur die Wunde sondern auch das Trennelement insgesamt. An dem Abdeckelement ist ein Anschlussventil vorgesehen, über welche eine Unterdruckquelle anschließbar ist. Mittels der Unterdruckquelle, etwa eine Pumpe, kann unterhalb des Abdeckelementes im Bereich der Wunde ein Unterdruck eingestellt werden. Hierdurch wird Wundsekret von dem verletzten Gewebe durch das Trennelement in den Absorptionskörper abgeführt. Von dem Absorptionselement kann das Wundsekret über das Anschlussventil nach außen abgeleitet werden.

Ein Trennelement ist für die erfindungsgemäße Verbandanordnung vorgesehen, wobei das Trennelement lösbar auf einem Trägerelement angeordnet ist. Das Trägerelement ist dabei eine dünne Kunststofffolie oder ein Papier, auf welche das Trennelement zunächst lösbar aufgebracht ist. Dies ermöglicht eine zuverlässige Handhabung des dünnen Trennelementes.

Dabei ist es nach einer Weiterbildung besonders bevorzugt, dass das Trägerelement eine zuschneidbare Trägerfolie ist. Somit kann das Trägerelement gemeinsam mit der Trägerfolie auf die Größe der Wunde zugeschnitten werden. Dies erlaubt ein sehr exaktes Zuschneiden des dünnen und damit sehr empfindlichen Trennelementes. Erst unmittelbar vor dem Auftragen des Trennelementes auf den Wundbereich wird das Trennelement von der Trägerfolie abgezogen.

Eine weitere Verbesserung der Handhabung wird nach einer erfindungsgemäßen Ausführung dadurch erreicht, dass das Trennelement und/oder die Trägerfolie transparent sind. Somit kann die Anordnung aus Trennelement und Trägerfolie unmittelbar auf den Wundbereich gehalten und dort gezielt auf die benötigte Größe zugeschnitten werden.

Weiterhin ist es besonders zweckmäßig, dass das Trennelement auf dem Trägerelement mit einer Haftschicht lösbar angebracht ist, welche zum Befestigen des Trennelementes auf einem Hautbereich ausgebildet ist. Die Haftschicht, welche vorzugsweise aus zwei gegenüberliegenden Haftstreifen entlang den Längskanten des Trennelementes besteht, kann somit eine doppelte Funktion erfüllen, nämlich die Befestigung auf dem Trägerelement sowie die Befestigung auf dem Hautbereich.

Ein Verfahren zum Behandeln einer Wunde ist dadurch gekennzeichnet, dass das Trennelement größer als die zu behandelnde Wunde ausgebildet wird, wobei ein überstehender Randabschnitt gebildet ist, und dass der Randabschnitt auf einen Hautbereich am Rand der Wunde aufgebracht wird. Das Verfahren wird insbesondere unter Verwendung einer Verbandanordnung durchgeführt, wie dies zuvor beschrieben worden ist. Es werden entsprechend die zuvor beschriebenen Vorteile erzielt.

Eine besonders gute Handhabung wird nach einer Verfahrensvariante dadurch erreicht, dass das Trennelement mit einer Haftschicht an dem Randabschnitt auf dem Hautbereich aufgeklebt wird. Die Haftschicht ist dabei vorzugsweise entlang von zwei Randseiten des Trennelementes angeordnet. Hierdurch kann ein zuverlässiges, jedoch nicht zu fest sitzendes Anbringen des Trennelementes auf der Wunde erzielt werden.

Eine weitere bevorzugte Ausführungsform des Verfahrens besteht darin, dass das Trennelement auf einem zuschneidbaren Trägerelement lösbar angebracht ist und vor dem Aufbringen auf die Wunde mit dem Trägerelement auf die vorgesehene Größe zugeschnitten wird. Dies erlaubt eine sehr gute Handhabung und ein besonders passgenaues Zuschneiden des dünnen und empfindlichen Trägerelementes gemeinsam mit dem stabilen Trägerelement. Das Trägerelement kann ein Papier, ein Gewebe oder vorzugsweise eine Kunststofffolie sein.

Die Erfindung wird nachfolgend anhand von bevorzugten Ausführungsbeispielen weiter beschrieben, die schematisch in den Zeichnungen dargestellt sind. In den Zeichnungen zeigen:
- Fig. 1: eine schematische Querschnittsansicht zu einer erfindungsgemäßen Verbandanordnung; und
- Fig. 2: eine schematische Draufsicht auf ein Trennelement gemäß der Erfindung.

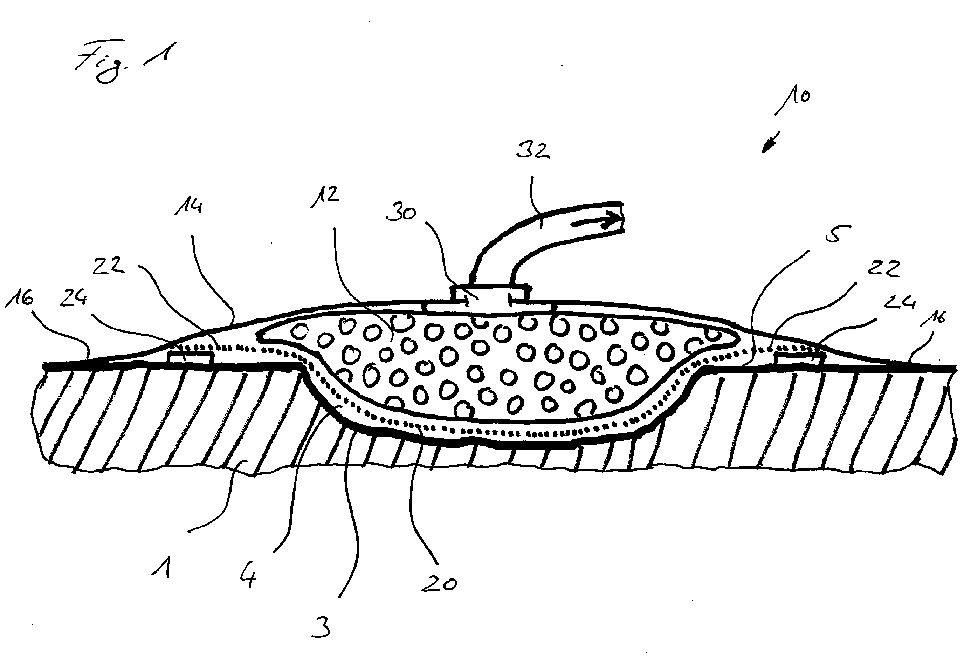

Gemäß Fig. 1 ist eine Wunde 3 in einem Körper 1 eines Menschen oder eines Tieres mit einem Hohlraum 4 dargestellt. Der Hohlraum 4 wird dabei im Wesentlichen durch einen eingesetzten Absorptionskörper 12 aus einem Schaumstoffmaterial ausgefüllt. Der Absorptionskörper 12 wird üblicherweise abhängig von der Größe der Wunde individuell zugeschnitten.

Um ein Einwachsen des Absorptionskörpers 12 in der Wunde 3 zu verhindern, ist zwischen dem Absorptionskörper 12 und der Wunde 3 ein Trennelement 20 aus einem Gewebe angeordnet. Gemäß der Erfindung erstreckt sich das dünne Trennelement 20 mit seitlichen Randabschnitten 22 über den eigentlichen Bereich der Wunde 3 hinaus und ist über zwei zueinander beabstandete, streifenförmige Haftschichten 24 an einem Hautbereich des Körpers 1 befestigt. Das Trennelement 20 wird dabei vor einem Einsetzen des Absorptionskörpers 12 in den Hohlraum 4 in die Wunde 3 eingelegt. Damit kann der Absorptionskörper 12 gefahrlos unmittelbar an der Wunde 3 zugeschnitten werden. Bei dem Zuschneiden des Schaumstoffmaterials des Absorptionskörpers 12 auftretende kleine Abfallpartikel können so durch das Trennelement 20 vor einem unmittelbaren Eintritt in die Wunde 3 zurückgehalten werden.

Das Trennelement 20 ragt definiert mit Randabschnitten 22 über die Wunde 3 hinaus, wobei das Trennelement 20 mittels Haftstreifen 24 an der Unterseite der Randabschnitte 22 an dem Hautbereich 5 des Körpers 1 befestigt werden kann. Dabei bilden die überstehenden Randabschnitte 22 einen zusätzlichen Schutz auch für den Hautbereich 5 entlang des Randes der Wunde 3. Hierdurch kann der Absorptionskörper 12 aus dem Schaumstoffmaterial auch aus der Wunde 3 in den Hautbereich 5 hineinragen, ohne dass eine merkliche Reizung der Haut oder eine schädliche Mazeration durch das Schaumstoffmaterial auftritt. Dies ermöglicht es auch, weitgehend vorgeschnittene Absorptionskörper 12 ohne ein individuelles Zuschneiden in Wunden 3 einzusetzen.

Der Absorptionskörper 12 und das definiert gegenüber der Wunde 3 und dem Absorptionskörper 12 vorstehende Trennelement 20 werden von einem folienartigen Abdeckelement 14 überdeckt. Zumindest entlang von Befestigungsabschnitten 16 ist das Abdeckelement 14 luftdicht an der Haut des Körpers 1 angebracht. Über eine ventilartige Anschlusseinrichtung 30 kann mittels einer Schlauchleitung 32 über eine nicht dargestellte Unterdruckquelle Luft aus dem abgedeckten Bereich der Wunde 3 abgeführt werden. Hierdurch wird in dem abgedeckten Wundbereich ein Unterdruck eingestellt. Durch diesen wird bewirkt, dass Wundsekret durch das gewebeartige Trennelement 20 hindurch in den Absorptionskörper 12 eintritt. Hierdurch kann das Wundsekret gesundes Körpergewebe nicht mehr schädigen und den Wundheilungsprozess behindern. Das Wundsekret kann im Absorptionskörper 12 verbleiben oder über die Schlauchleitung 32 insgesamt aus dem abgedeckten Wundbereich abgeführt werden.

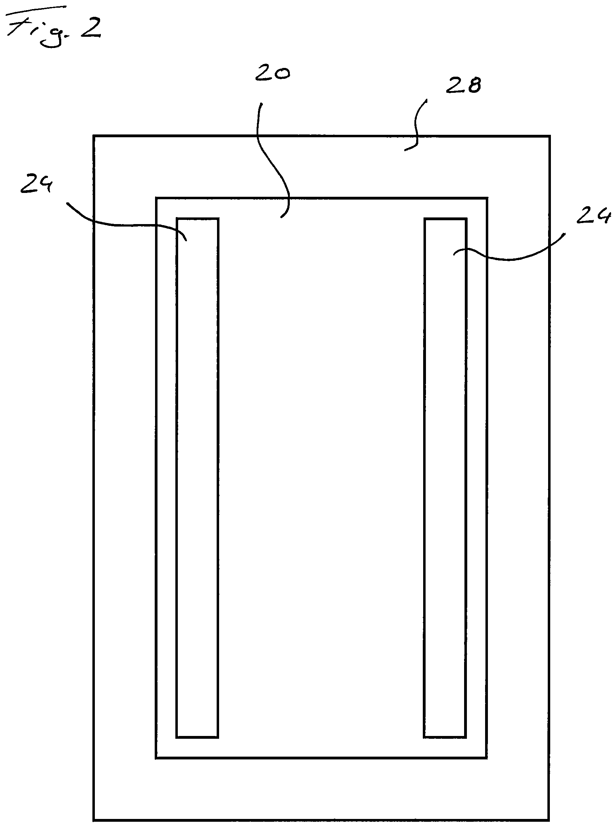

Ein Ausführungsbeispiel eines Trennelementes 20 nach der Erfindung ist in Fig. 2 dargestellt. Das Trennelement 20, welches vorzugsweise ein Gewebe ist, ist dabei mittels zwei streifenförmigen Haftschichten 24 entlang den Längsseiten des Trennelementes 20 auf einem folienartigen Trägerelement 28 aufgebracht. Das Trennelement 20 und das Trägerelement 28 sind dabei im Wesentlichen transparent ausgebildet, so dass es unmittelbar im Bereich der Wunde bedarfsgerecht zugeschnitten werden kann. Das dünne Trennelement 20 und das ebenfalls dünne Trägerelement 28 können gemeinsam mit einer üblichen Schere maßgenau zugeschnitten werden. Nach dem Zuschneiden kann das dünne und empfindliche Trennelement 20 von dem folienartigen Trägerelement 28 abgezogen werden, wobei die Haftschichten 24 an dem Trennelement 20 verbleiben. Das Trennelement 20 kann sodann mit den Haftschichten 24 auf den Wundbereich aufgebracht werden, wobei die Haftschichten 24 zum Befestigen an dem Hautbereich 5 am Rand der Wunde 3 dienen.

## Patentansprüche

1. Verbandanordnung zum Behandeln einer Wunde (3) eines menschlichen oder tierischen Körpers (1) mittels Unterdruck, mit
- einem Absorptionskörper (12) zum Absorbieren von Wundsekret, wobei der Absorptionskörper (12) zum Einsetzen in einen Hohlraum (4) der Wunde (3) ausgebildet ist,
- einem Trennelement (20), welches zwischen der Wunde (3) und dem Absorptionskörper (12) angeordnet ist, wobei das Trennelement (20) ein Gewebe umfasst,
- einem Abdeckelement (14), welches zum weitgehend luftdichten Abdecken der Wunde (3) mit dem Absorptionskörper (12) ausgebildet ist, und
- einer Anschlusseinrichtung (30), welche zum Anschließen einer Unterdruckquelle an dem Abdeckelement (14) angeordnet ist,
- das Trennelement (20) größer als die zu behandelnde Wunde (3) ausgebildet ist, wobei ein überstehender Randabschnitt (22) gebildet ist, und
- der Randabschnitt (22) an einem Hautbereich (5) am Rand der Wunde (3) angeordnet ist,
**dadurch gekennzeichnet,**
- **dass** das Gewebe aus Monofilamentfäden mit einer glatten Oberfläche gebildet ist, welche einen Fadendurchmesser zwischen 10 bis 100 µm aufweisen,
- **dass** das Trennelement (20) biokompatibel und antihaftend ausgebildet ist, und
- **dass** das Gewebe mit einer Maschenweite von 40 bis 500 µm vorgesehen ist.

2. Verbandanordnung nach Anspruch 1,
**dadurch gekennzeichnet,**
**dass** an dem Randabschnitt (22) zumindest bereichsweise eine Haftschicht (24) zum Ankleben des Trennelementes (20) auf den Hautbereich (5) angeordnet sind.

3. Verbandanordnung nach Anspruch 1 oder 2,
**dadurch gekennzeichnet,**
**dass** die Monofilamentfäden einen Fadendurchmesser zwischen 30 bis 50 µm aufweisen.

4. Verbandanordnung nach einem der Ansprüche 1 bis 3,
**dadurch gekennzeichnet,**
**dass** das Gewebe mit einer Maschenweite von 70 bis 300 µm vorgesehen ist.

5. Verbandanordnung nach einem der Ansprüche 1 bis 4,
**dadurch gekennzeichnet,**
**dass** das Abdeckelement (14) größer als das Trennelement (20) ausgebildet ist.

6. Verbandanordnung nach einem der Ansprüche 1 bis 5,
**dadurch gekennzeichnet,**
**dass** das Trennelement (20) lösbar auf einem Trägerelement (28) angeordnet ist.

7. Verbandanordnung nach Anspruch 6,
**dadurch gekennzeichnet,**
**dass** das Trägerelement (28) eine zuschneidbare Trägerfolie ist.

8. Verbandanordnung nach Anspruch 6 oder 7,
**dadurch gekennzeichnet,**
**dass** das Trennelement (20) und/oder das Trägerelement (28) transparent sind.

9. Verbandanordnung nach einem der Ansprüche 6 bis 8,
**dadurch gekennzeichnet,**
**dass** das Trennelement (20) auf dem Trägerelement (28) mit einer Haftschicht (24) lösbar angebracht ist, welche zum Befestigen des Trennelementes (20) auf einem Hautbereich (5) ausgebildet ist.

## Claims

1. Dressing arrangement for treating a wound (3) of a human or animal body (1) by means of negative pressure, having
- an absorption body (12) for absorbing wound secretion, wherein the absorption body (12) is designed for insertion into a cavity (4) of the wound (3),
- a separation element (20) which is arranged between the wound (3) and the absorption body (12), wherein the separation element (20) comprises a fabric,
- a cover element (14) which is designed for substantially airtight covering of the wound (3) with the absorption body (12), and
- a connection means (30) which is arranged on the cover element (14) for connection of a negative pressure source,
- the separation element (20) is designed larger than the wound (3) to be treated, wherein a protruding edge section (22) is formed, and
- the edge section (22) is arranged on a skin region (5) on the edge of the wound (3),
**characterized in that**
- the fabric is formed of monofilament threads with a smooth surface which have a thread diameter ranging between 10 and 100 µm,
- **in that** the separation element (20) is of biocompatible and anti-adhesive design and
- **in that** the fabric is provided with a mesh width ranging from 40 to 500 µm.

2. Dressing arrangement according to claim 1,
**characterized in that**
on the edge section (22) an adhesive layer (24) is arranged at least in some regions to adhere the separation element (20) onto the skin region (5).

3. Dressing arrangement according to claim 1 or 2,
**characterized in that**
the monofilament threads have a thread diameter ranging between 30 and 50 µm.

4. Dressing arrangement according to any one of claims 1 to 3,
**characterized in that**
the fabric is provided with a mesh width ranging from 70 to 300 µm.

5. Dressing arrangement according to any one of claims 1 to 4,
**characterized in that**
the cover element (14) is designed larger than the separation element (20).

6. Dressing arrangement according to any one of claims 1 to 5,
**characterized in that**
the separation element (20) is releasably arranged on a carrier element (28).

7. Dressing arrangement according to claim 6,
**characterized in that**
the carrier element (28) is a carrier film that can be cut.

8. Dressing arrangement according to claim 6 or 7,
**characterized in that**
the separation element (20) and/or the carrier element (28) are transparent.

9. Dressing arrangement according to any one of claims 6 to 8,
**characterized in that**
the separation element (20) is releasably attached to the carrier element (28) by means of an adhesive layer (24) which is designed for fixing the separation element (20) on a skin region (5).

## Revendications

1. Système de pansement pour le traitement d'une plaie (3) d'un corps humain ou animal (1) par dépression, comportant :
- un corps d'absorption (12) pour l'absorption d'une sécrétion de plaie, le corps d'absorption (12) étant réalisé pour l'insertion dans un espace creux (4) de la plaie (3),
- un élément de séparation (20) qui est agencé entre la plaie (3) et le corps d'absorption (12), l'élément de séparation (20) comprenant un tissu,
- un élément de recouvrement (14) qui est réalisé pour un recouvrement largement étanche à l'air de la plaie (3) avec le corps d'absorption (12), et
- un dispositif de raccordement (30) qui est agencé pour le raccordement d'une source de dépression à l'élément de recouvrement (14),
- l'élément de séparation (20) est réalisé plus grand que la plaie (3) à traiter, une section de bord (22) en saillie étant formée, et
- la section de bord (22) est agencée au niveau d'une zone de peau (5) au niveau du bord de la plaie (3),
**caractérisé en ce**
- **que** le tissu est formé de fils monofilament avec une surface lisse qui présentent un diamètre de fil entre 10 et 100 µm,
- **que** l'élément de séparation (20) est réalisé de manière biocompatible et antiadhésive, et
- **que** le tissu est prévu avec une largeur de maille de 40 à 500 µm.

2. Système de pansement selon la revendication 1,
**caractérisé en ce**
**qu'**une couche adhésive (24) est agencée pour le collage de l'élément de séparation (20) sur la zone de peau (5) au moins par endroits au niveau de la section de bord (22).

3. Système de pansement selon la revendication 1 ou 2,
**caractérisé en ce**
**que** les fils monofilament présentent un diamètre de fil entre 30 et 50 µm.

4. Système de pansement selon l'une des revendications 1 à 3,
**caractérisé en ce**
**que** le tissu est prévu avec une largeur de maille de 70 à 300 µm.

5. Système de pansement selon l'une des revendications 1 à 4,
**caractérisé en ce**
**que** l'élément de recouvrement (14) est réalisé plus grand que l'élément de séparation (20).

6. Système de pansement selon l'une des revendications 1 à 5,
**caractérisé en ce**
**que** l'élément de séparation (20) est agencé de manière amovible sur un élément de support (28).

7. Système de pansement selon la revendication 6,
**caractérisé en ce**
**que** l'élément de support (28) est un film de support découpable.

8. Système de pansement selon la revendication 6 ou 7,
**caractérisé en ce**
**que** l'élément de séparation (20) et/ou l'élément de support (28) sont transparents.

9. Système de pansement selon l'une des revendications 6 à 8,
**caractérisé en ce**
**que** l'élément de séparation (20) est monté de manière amovible sur l'élément de support (28) avec une couche adhésive (24) qui est réalisée pour la fixation de l'élément de séparation (20) sur une zone de peau (5).
